# EUROPEAN PATENT APPLICATION

(11) **EP 3 281 957 A1**
(43) Date of publication of application: **14.02.2018**
(21) Application number: 16306057.7
(22) Date of filing: 13.08.2016
(51) Int. Cl.: C08B 37/16

(54) **AZA-CAPPED CYCLODEXTRINS AND PROCESS OF PREPARING THEM**

(71) Applicant: Université de Strasbourg, 67000 Strasbourg (FR); Centre National de la Recherche Scientifique, 75016 Paris (FR)
(72) Inventor: MATT, Dominique, 67000 STRASBOURG (FR); ARMSPACH, Dominique, 67000 STRASBOURG (FR); JOUFFROY, Matthieu, RAHWAY, NJ New Jersey NJ 07065 (US)
(74) Representative: Pontet Allano & Associes

(57) **Abstract**

Rigidified cyclodextrin derivatives comprising a core formed of a cyclodextrin unit wherein two glucose units, whether adjacent or not, are linked through a -N(R¹R²)- bridge attached to the C-6 carbon atoms of the bridged glucose units, the C-6 carbon atom of glucose units which are not bound to the said bridge are substituted by R³ residues, and the C-2 and C-3 carbon atoms are substituted by OR⁴ residues, the said derivative being of formula (I) 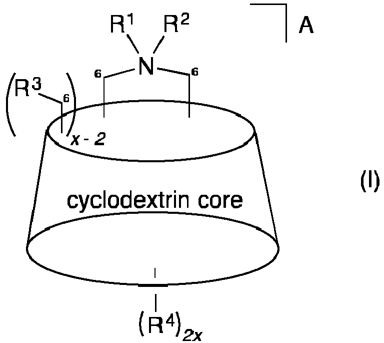 wherein "cyclodextrin core" represents the moiety obtained by removal of all -CH₂OH groups and all hydrogen atoms of the hydroxyl groups attached to the C-2 and C-3 carbon atoms of the glucose units of alpha-cyclodextrin, beta-cyclodextrin or gamma-cyclodextrin, R¹ and R² are chosen from hydrogen and carbon-based radicals, the R³ residues are chosen from hydrogen, the hydroxyl and carbon-based radicals, the R⁴ residues are chosen from hydrogen and carbon-based radicals and A is an anion or a mixture of anions.

## Description

The invention relates to aza-capped cyclodextrins, their synthesis and their use in several fields.

Cyclodextrins are naturally occurring cyclic oligosaccharides. The three commercially available native cyclodextrins, namely alpha-, beta- and gamma-cyclodextrins, are respectively made up of 6, 7, and 8 alpha(1-> 4)-linked D-(+)-glucopyranose units. They possess a hollow truncated cone structure bearing hydroxymethyl groups at the narrower end, and secondary 2- and 3-hydroxyl groups at their wider end. They may be represented by the following formula (CD).

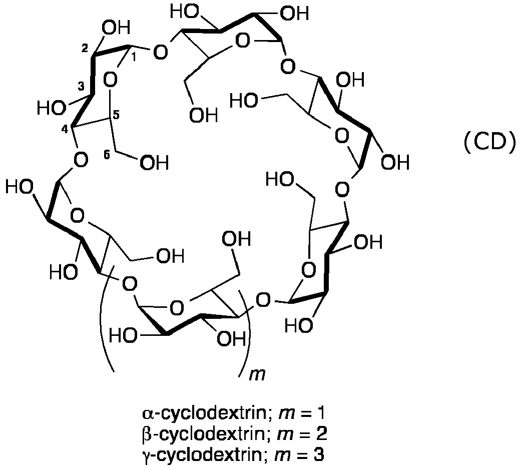

The presence of a relatively hydrophobic cavity is responsible for their capacity to form inclusion complexes in water with numerous lipophilic molecules, in particular biologically active ones. Because of these particular host-guest properties, they are extensively used as food, cosmetics (E. Bilensoy, Cyclodextrins in Pharmaceutics, Cosmetics and Biomedicine, John Wiley & Sons, New Jersey, 2011), drug (E. Bilensoy previously cited) and textile additives (U. R. Bhaskara-Amrit, P. et al., Autex Res. J., 2011, 11, 94-101), catalysts in chemical and industrial processes (H. Bricout et al., Curr. Org. Chem., 2010, 14, 1296-1307 and F. Hapiot et al., C. R. Chim., 2011, 14, 149-166) and in separation science and technology (Y. Xiao et al., C. R. Chim., 2011, 14, 149-166). When trapped in the cavity of a cyclodextrin or one of its derivatives, the physical and chemical environment of a given compound is altered so that reactions involving this compound are affected by the inner-cavity environment. In particular, because of the chiral nature of cyclodextrins and their derivatives, chiral induction is usually observed in otherwise non-asymmetric reactions. Aminodeoxy cyclodextrin derivatives (I. Tabushi et al., Am. Chem. Soc., 1979, 101, 4759-4760), especially when comprising other donor groups, as well as quaternary ammonium-bearing cyclodextrin derivatives, are of particular interest for their properties in asymmetric catalysis (Y. Matsui et al., Bull. Chem. Soc. Jpn., 1978, 51, 3030-3034 and W. Tagaki, et al., Tetrahedron Lett., 1990, 31, 3897-3900) and their capacity to act as chiral selectors (H. Parrot-Lopez et al., Tetrahedron-Asymmetr., 1990, 1, 367-370), notably in capillary electrophoresis (O. A. Shpigun, et al., Russ. Chem. Rev., 2003, 72, 1035-1054).

A number of studies dealing with compounds having a simply or doubly capped cyclodextrin scaffold, in particular phosphanes, thioethers and sulfates have been reported (R. Gramage-Doria et al., Chem.Eur. J., 2011, 17, 3911-3921). Such derivatives are cavity-shaped ligands which are useful in metal complexation and catalysis (Gramage-Doria et al., Dalton Trans, 2012, 41, 8786-8795 ; Chem. Eur. J., 2012, 18, 10813-10816) and as catalysts (M. Jouffroy et al., Angew. Chem., 2014, 3937-3940; Beilstein J. Org. Chem., 2014, 10, 2388-2405).

The synthesis of a number of cyclodextrin derivatives with amino and quaternary ammonium units has already been described (for example in US 3,453,257; US 3,553,191; EP 0387 681 and US 2005/0059634). In all these compounds, the nitrogen atom(s) are part of dangling and thus flexible substituent(s) in which the nitrogen atom(s) is (are) not spatially fixed with respect to the cavity of the cyclodextrin derivative. The high mobility of the nitrogen atoms in these compounds may restrict the capacity of the nitrogen atom to act synergistically with the receptor unit of the cyclodextrin derivative.

Thus there is still a need to provide cyclodextrin derivatives having their nitrogen atoms(s) rigidly positioned with respect to the hydrophobic cyclodextrin receptor. This need is satisfied by the cyclodextrin derivatives according to the invention, which bear *N*-organylamino (-NR¹-) or *N,N-*diorganylamino (-NR¹R²-) diradical bridges, or a combination thereof, which link two glucose units of the cyclodextrin core through C-6 carbon atoms (interchangeably noted C-6 or C⁶ hereafter), the said glucose units being adjacent or not.

Thus a first object of the invention is rigidified cyclodextrin derivatives comprising a core formed of a cyclodextrin unit wherein
a. two glucose units, whether adjacent or not, are linked through a -N(R¹R²)- bridge attached to the C-6 carbon atoms of the bridged glucose units,
b. the C-6 carbon atom of glucose units which are not bound to the said bridge are substituted by R³ residues, and
c. the C-2 and C-3 carbon atoms are substituted by OR⁴ residues,
the said derivative being of formula (I) 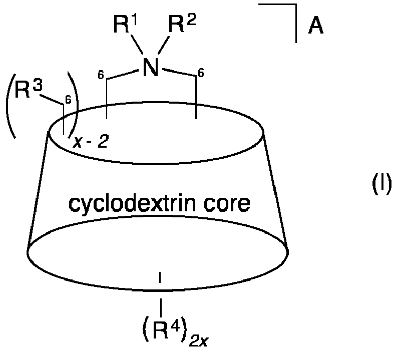 wherein,
"cyclodextrin core" represents the moiety obtained by removal of all -CH₂OH groups and all hydrogen atoms of the hydroxyl groups attached to the C-2 and C-3 carbon atoms of the glucose units of alpha-cyclodextrin, beta-cyclodextrin or gamma-cyclodextrin responding to the following formula, 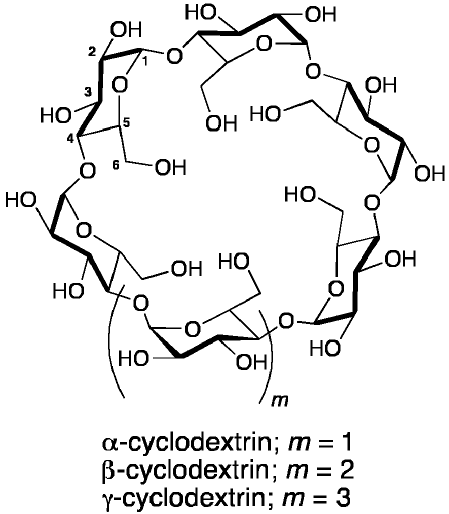
x is an integer comprised between 6 and 8,
R¹, optionally linked to R², is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
R², optionally absent, is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
the R³ residues, identical or different, optionally linked together or linked to the R¹ or R² residues, are chosen from hydrogen, the hydroxyl radical, and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
R⁴, identical or different, optionally linked together, are chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
A, is an anion, preferentially selected from Cl⁻, Br, I⁻, BF₄⁻, PF₆⁻, B(aryl)₄⁻ CF₃SO₃⁻, AcO⁻, CF₃CO₂⁻, PO₄⁻, SO₄⁻, picrate, phosphonate, or a mixture thereof, the said anion or mixture of anions having a total charge ranging from 0 to 20, preferentially from 0 to 4,
with the proviso that the compounds of formula (a) are excluded

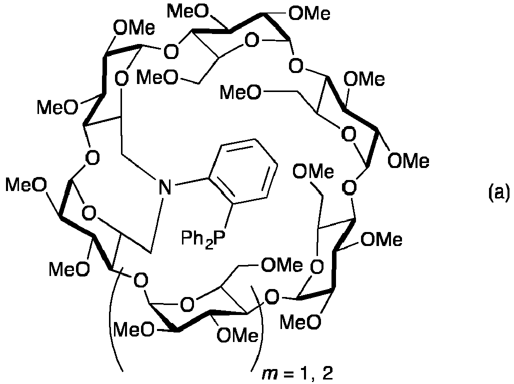

According to the invention, when the saturated or unsaturated, optionally aromatic, linear, branched or cyclic C₁-C₁₀₀ carbon-based radicals comprise heteroatoms, the said heteroatoms may be part of the chain of the said radicals or of their substituents or of both. Advantageously, the saturated or unsaturated, optionally aromatic, linear, branched or cyclic carbon-based radicals are C₁-C₁₀₀ carbon based radicals.

As typical examples of such carbon-based radicals may be cited: (C₁-C₂₈)alkyl groups, (C₁-C₂₈)alkylsulfonate groups, (C₁-C₂₈)alkoxy groups, (C₃-C₂₈)cycloalkyl groups, aromatic groups like phenyl, naphthyl, pyridyl, imidazolyl, thienyl or cyclodextrin radicals. In particular, R¹ may be hydrogen, a methyl group, a 2-nitrosulfonyl group or a 2-pyridylmethyl group, R² may be a methyl group, R³ may be a methoxy group or a methylsulfonyloxy group and R⁴ may be a methyl group.

According to the invention, when at least two R³ residues are linked together or when at least one R³ is linked to the R¹ or R² residues, then the cyclodextrin derivative has several bridges. The same holds when at least two R⁴ substituents are linked together. All these bridges may contain a heteroatom. Thus, these particular cyclodextrin derivatives may have two glucose units, whether adjacent or not, linked through a bridge formed by a -N(R¹R²)-moiety attached to the C-6 carbon atoms of the bridged glucose units and at least two other glucose units, whether adjacent or not, linked through a bridge formed by a moiety containing a heteroatom attached to the C-6 carbon atoms of the bridged glucose units.

The compounds according to the invention may be prepared according to new processes relying on state-of-the-art reactions and using commercially available compounds or with compounds prepared according to known processes. The inventors provide for the first time a process to prepare well-defined cylodextrin derivatives with nitrogen-bridged glucose units.

Therefore a second object of the present invention is a process for the preparation of derivatives of formula (I) comprising the following steps:
a) reacting a cyclodextrin derivative of formula II: 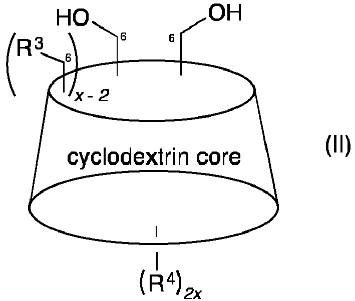
   wherein R³, R⁴ and x are as defined above,
   with 2-nitrobenzenesulfonamide under Mitsonobu conditions, so as to obtain the cyclodextrin derivative of formula (I) in which R¹ is a 2-nitrobenzenesulfonyl group and in which R² is absent,
b) hydrolyzing the sulfonyl derivative obtained in step a) to yield a derivative of formula (I) wherein R¹ is H and wherein R² is absent,
c) reacting the derivative obtained in step b), in the presence of a reducing agent, preferably sodium cyanoborohydride, with an aldehyde of formula (III)

   R⁵-CHO (III)

   in which R⁵ is chosen from hydrogen and saturated or unsaturated, optionally aromatic, linear, branched or cyclic C₁-C₁₀₀ carbon-based radicals, optionally comprising 1 to 50 heteroatoms chosen from O, N, S, F, Cl, Br, I, Si, and P,
   to afford a tertiary amine of formula (I) wherein R¹ is defined as above and where R² is absent.

A third object of the invention is a process for the preparation of derivatives of formula (I) comprising the step of reacting a cyclodextrin derivative of formula IV: 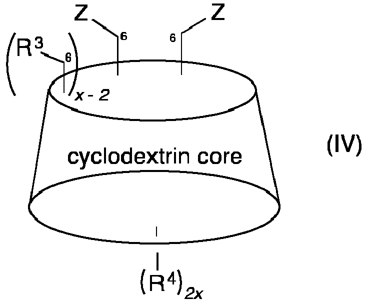 wherein R³, R⁴ and x are as defined above, and wherein the Z residues, identical or different and attached to C-6 carbon atoms of either adjacent or non adjacent glucose units, are leaving groups, selected from the group comprising *p*-toluene sulfonyloxy, methylsulfonyloxy, trifluoromethylsulfonyloxy, chloro, bromo or iodo,
with 2-nitrobenzenesulfonamide in the presence of a carbonate, preferentially selected from the group comprising Li₂CO₃, Na₂CO₃, K₂CO₃, and Cs₂CO₃, so as to obtain the cyclodextrin derivative of formula (I), wherein R¹ is the 2-nitrobenzenesulfonyl group, and R² is absent.

A fourth object of the invention is a process comprising the step of reacting a derivative of formula (I), wherein R¹, R³, R⁴, A, x and y are as defined above, and wherein R² is absent,
with a R²X reagent, wherein R² is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
and wherein X is a leaving group, selected from *p*-toluene sulfonyloxy, methylsulfonyloxy, trifluoromethylsulfonyloxy, chloro, bromo or iodo to obtain a derivative of formula (V) 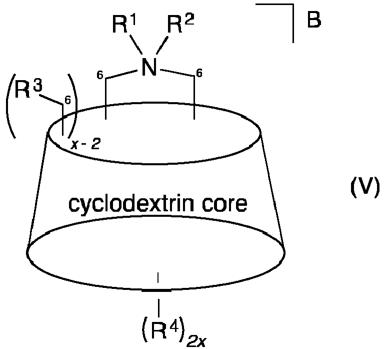 wherein R¹, R², R³, R⁴, x and y are as defined as above and B is an anion or a mixture of anions of a total charge superior to that of A.

The cyclodextrin derivatives according to the invention bearing amines can be used as ligands for metal complexation and catalysis, whereas those containing ammonium subunits can be used as chiral supramolecular phase transfer catalysts, chiral selectors in capillary electrophoresis, or auxiliaries for the resolution of racemic anions.

Applications of these compounds include also their use as chiral components of stationary phases for chromatographic purposes, anti-bleaching agents for textiles, food and drug additives, pharmaceuticals, bioactive compounds, asymmetric catalysts, chiral ligands, chiral selectors in analytical and preparative procedures.

Thus a fifth object of the invention is a separation material for application in chromatography comprising a rigidified cyclodextrin derivative according to the invention grafted on a support.

A sixth object of the invention is a method for protecting colored cellulosic fibers involving grafting of derivatives according to the invention on the said fibers.

A seventh object of the invention is a complex for targeted drug delivery comprising a rigidified cyclodextrin derivative according to the invention and a therapeutic agent.

The following examples illustrate the synthesis of compounds according to the invention.

### Example 1: Preparation of 6^{A},6^{B}-dideoxy-6^{A},6^{B}-N-((2-nitrophenyl)sulfonyl)aza-2^{A},2^{B},2^{C},2^{D},2^{E},2^{F},3^{A},3^{B},3^{C},3^{D},3^{E},3^{F},6^{C},6^{D}, 6^{E},6^{F}-hexadeca-O-methyl-alpha-cyclodextrin

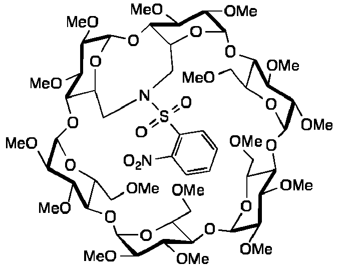

### 1.1. Method 1

To a solution of 2-nitrobenzenesulfonamide (83 mg, 0.41 mmol) and 2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (397 mg, 0.33 mmol) in dry toluene (5 mL), were added simultaneously and dropwise a solution of triphenylphosphine (PPh₃) (349 mg, 1.33 mmol) in dry toluene (4mL) and a solution of diisopropyl azodicarboxylate (DIAD) (269 mg, 262 µL, 1.33 mmol) in dry toluene (4 mL) over a period of 45 min at room temperature. The yellow solution was stirred under nitrogen for 3 h. If necessary, extra PPh₃ (86 mg, 0.33 mmol) and DIAD (67 mg, 65 µL, 0.33 mmol) were added to complete the reaction. The suspension was then evaporated to dryness. Finally, the crude was subjected to column chromatography (SiO₂; CH₂Cl₂/MeOH, 97:3, *v*/*v*) to afford 6^{A},6^{B}-dideoxy-6^{A},6^{B}-*N*-((2-nitrophenyl)sulfonyl)aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (277 mg, 61%) as a colorless solid. *R*_{f} (SiO₂, CH₂Cl₂/MeOH, 90:10, *v*/*v)* = 0.45;
¹H NMR (400.1 MHz, CDCl₃, 25°C): *δ* (assignment by COSY) = 3.01 (dd, 1 H, ²*J*_{H6a-H6b} = 13.0 Hz, ³*J*_{H6a-H5} = 9.4 Hz, H-6a^{A or B}), 3.09-3.22 (6 H, H-2, H-4), 3.34 (s, 3 H, OMe), 3.38 (s, 3 H, OMe), 3.40 (s, 3 H, OMe), 3.44 (s, 3 H, OMe), 3.46 (s, 3 H, OMe), 3.47 (s, 3 H, OMe), 3.49 (s, 3 H, OMe), 3.51 (s, 3 H, OMe), 3.52 (s, 3 H, OMe), 3.59 (s, 3 H, OMe), 3.62 (s, 3 H, OMe), 3.63 (s, 3 H, OMe), 3.65 (s, 6 H, OMe), 3.70 (s, 3 H, OMe), 3.30-3.99 (25 H, H-2, H-3, H-4, H-5, H-6), 4.15 (d, 1 H, ²*J*_{H6b-H6a} = 13.0 Hz, H-6b^{A or B}), 4.22-4.31 (2 H, H-5^{A or B}), 4.34 (d, 1 H, ²*J*_{H6b-H6a} = 12.0 Hz, H-6), 4.99-5.03 (4 H, H-1), 5.08 (d, 1 H, ³*J*_{H1-H2} = 3.2 Hz, H-1), 5.10 (d, 1 H, ³*J*_{H1-H2} = 3.3 Hz, H-1), 7.63-7.75 (4 H, aromatic-H) ppm; ¹³C{¹H} NMR (100.6 MHz, CDCl₃, 25°C): *δ* (assignment by combined HSQC and HMBC) = 49.72, 54.63 (C-6^{A,B}), 57.44, 57.61, 57.74, 57.94 [x2], 58.66, 58.83 [x2], 59.02, 59.17, 60.86, 61.53, 61.65, 64.75 [×3] (OMe), 69.32 (C-5), 70.33, 70.44 (C-6), 70.84 [x2], 70.91, (C-5), 71.07, 71.26 (C-6), 71.70, 75.03 (C-5), 80.15, 81.07, 81.17, 81.24, 81.35 [x4], 81.76, 81.83, 81.90 [x2], 81.95, 82.16 [×2], 82.55, 83.71, 86.16 (C-2, C-3, C-4), 96.88, 97.38, 99.61, 100.27, 100.45, 100.81 (C-1), 124.49, 128.59, 131.38, 132.64, 133.35, 148.61 (C aromatic) ppm;
elemental analysis (%) calcd for C₅₈H₉₄N₂O₃₂S·CH₂Cl₂ (1363.43 + 84.93): C 48.93, H 6.68, N 1.93 found: C 49.29, H 6.66, N 1.99;
MS (ESI-TOF): *m*/*z* (%): 1385.54 (100) *[M* + Na]⁺.

### 1.2. Method 2

Potassium carbonate (30 mg, 0.22 mmol) was added to a solution of 6^{A},6^{B}-di-*O*-(methylsulfonyl)-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (100 mg, 0.074 mmol) and 2-nitrobenzenesulfonamide (15 mg, 0.074 mmol) in anhydrous dimethylformamide (2mL). The reaction mixture was evaporated to dryness *in vacuo* and the residue retaken in chloroform (100 mL). The organic solution was washed twice with distilled water (2 x 100 mL) and once with saturated NaCl solution (50 mL) before being dried (MgSO₄) and evaporated to dryness *in vacuo* to afford a yellow residue, which was subjected to column chromatography (SiO₂, CH₂Cl₂/MeOH, 97:3) to give 6^{A},6^{B}-dideoxy-6^{A},6^{B}-*N*-((2-nitrophenyl)sulfonyl)aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-O-methyl-alpha-cyclodextrin (89 mg, 88 %) as a colorless solid.

### Example 2: Preparation of 6^{A},6^{B}-dideoxy-6^{A},6^{B}-N-((2-nitrophenyl)sulfonyl)aza-2^{A},2^{B},2^{C},2^{D},2^{E},2^{F},2^{G},3^{A},3^{B},3^{C},3^{D},3^{E},3^{F},3^{G},6^{C},6^{D},6^{E},6^{F},6^{G}-hexadeca-O-methyl-beta-cyclodextrin

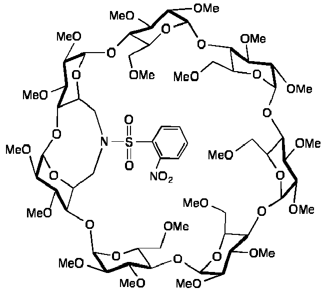

6^{A},6^{B}-dideoxy-6^{A},6^{B}-(*N*-(2-nitrophenyl)sulfonyl)-aza-2^{A},2^{B},2^{C},2^{D},2^{E}, 2^{F},2^{G},3^{A},3^{B},3^{C},3^{D},3^{E},3^{F},3^{G},6^{C},6^{D},6^{E},6^{F},6^{G}-hexadeca-*O*-methyl-beta-cyclodex-trin (colorless solid, 135 mg, 60%) was synthesized from 2^{A},2^{B},2^{C},2^{D},2^{E},2^{F},2^{G},3^{A}3^{B},3^{C},3^{D},3^{E},3^{F},3^{G},6^{C},6^{D},6^{E},6^{F},6^{G}-nonadeca-*O-*methyl-beta-cyclodextrin (200 mg, 0.14 mmol), 2-nitrobenzenesulfonamide (36 mg, 0.18 mmol), PPh₃ (150 mg, 0.57 mmol) and DIAD (115 mg, 112 µL, 0.57 mmol) in toluene (2.5 mL) according to method 1 in example 1.
*R*_{f} (SiO₂, CH₂Cl₂/MeOH, 90:10, *v*/*v)* = 0.41;
¹H NMR (500.1 MHz, CDCl₃, 25°C): *δ* (assignment by COSY) = 3.01 (dd, 1 H, ²*J*_{H6a-H6b} = 13.0 Hz, ³*J*_{H6a-H5} = 9.2 Hz, H-6a^{A or B}), 3.10 (dd, 1 H, ³*J*_{H2-H3} = 10.0 Hz, ³*J*_{H2-H1} = 3.0 Hz, H-2), 3.13 (t, 1 H, ³*J*_{H4-H5} = ³*J*_{H3-H4} = 9.7 Hz, H-4), 3.15-3.22 (5 H, H-2), 3.20 (s, 3 H, OMe), 3.26 (s, 3 H, OMe), 3.37 (s, 3 H, OMe), 3.40 (s, 3 H, OMe), 3.45 (s, 3 H, OMe), 3.47 (s, 3 H, OMe), 3.48 (s, 3 H, OMe), 3.49 (s, 3 H, OMe), 3.51 (s, 6 H, OMe), 3.52 (s, 3 H, OMe), 3.53 (s, 3 H, OMe), 3.58 (s, 3 H, OMe), 3.60 (s, 3 H, OMe), 3.62 (s, 3 H, OMe), 3.63 (s, 3 H, OMe), 3.65 (s, 3 H, OMe), 3.69 (s, 3 H, OMe), 3.70 (s, 3 H, OMe), 3.30-3.89 (27 H, H-2, H-3, H-4, H-5, H-6), 3.89-3.99 (3 H, H-6), 4.17 (d, 1 H, ³*J*_{H5-H6} = 9.3 Hz, H-5), 4.18 (dd, 1 H, ³*J*_{H5-H6b} = 19.7 Hz, ³*J*_{H5-H6a} = 9.8 Hz, H-5), 4.28 (d, 1 H, ²*J*_{H6b-H6a}, 13.0 Hz, H-6b^{A or B}), 4.32 (d, 1 H, ³*J*_{H6a-H6b} = 10.5 Hz, H-6), 4.99 (d, 1 H, ³*J*_{H1-H2} = 4.8 Hz, H-1), 5.05-5.07 (2 H, H-1), 5.08 (d, 1 H, ³*J*_{H1-H2} = 3.1 Hz, H-1), 5.12 (d, 1 H, ³*J*_{H1-H2} = 3.6 Hz, H-1), 5.13 (d, 1 H, ³*J*_{H1-H2} = 3.4 Hz, H-1), 5.34 (d, 1 H, ³*J*_{H1-H2} = 3.6 Hz, H-1), 7.63-7.68 (2 H, aromatic-H), 7.68-7.75 (m, 1 H, aromatic-H), 7.86-7.91 (m, 1 H, aromatic-H) ppm;
¹³C{¹H} NMR (125.8 MHz, CDCl₃, 25°C): *δ* (assignment by combined HSQC and HMBC) = 49.19, 54.34 (C-6^{A,B}), 57.73, 57.75, 58.18, 58.29, 58.46, 58.81 [x2], 58.96 [x2], 59.02 [x2], 59.24, 60.48, 61.13, 61.43, 61.61, 61.67 [x2], 61.75 (OMe), 69.42, 69.77, 70.49 (C-5), 70.65, 70.67, 70.74 (C-6), 70.80 (C-5), 71.02 [x2] (C-6), 71.31, 75.49, 75.72 (C-5), 79.78, 79.82, 80.16, 80,31, 80.50, 80.62, 81.26 [x2], 81.66 [x3], 81.71 [x3], 81.81, 81.88, 82.04, 82.44, 82.64, 84.93, 86.37 (C-2, C-3, C-4), 96.30, 97.71 [x2], 98.36, 98.45, 98.67, 100.32 (C-1), 124.31, 130.16, 131.05, 132.11, 133.50, 148.37 (C aromatic) ppm;
elemental analysis (%) calcd for C₆₇H₁₁₀N₂O₃₇S (1567,65) : C 51.33, H 7.07, N 1.79 found C 51.24, H 7.14, N 1.94;
MS (ESI-TOF): *m*/*z* (%): 1584.69 (100) *[M* + H₃O]⁺.

### EXAMPLE 3: Preparation of 6^{A},6^{B}-dideoxy-6^{A},6^{B}-N-((2-nitrophenyl)sulfonyl)-aza-6^{D},6^{E}-di-O-(methylsulfonyl)-2^{A},2^{B},2^{C},2^{D},2^{E},2^{F},3^{A},3^{B},3^{C},3^{D},3^{E},3^{F},6^{C},6^{D},6^{E},6^{F}-tetradeca-O-methyl-alpha-cyclodextrin

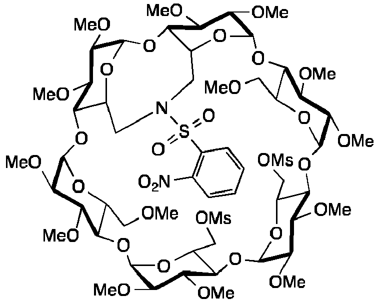

Potassium carbonate (120 mg, 0.87 mmol) was added to a solution of 6^{A},6^{B},6^{D},6^{E}-tetra-*O*-(methylsulfonyl)-2^{A},2^{B},2^{C},2^{D},2^{E},2^{F},3^{A},3^{B},3^{C},3^{D},3^{E},3^{F}, 6^{C},6^{D},6^{E},6^{F}-tetradeca-*O*-methyl-alpha-cyclodextrin (215 mg, 0.145 mmol) and 2-nitrobenzenesulfonamide (65 mg, 0.32 mmol) in anhydrous dimethylformamide (4 mL). The reaction mixture was evaporated to dryness *in vacuo* and the residue retaken in chloroform (100 mL). The organic solution was washed twice with distilled water (2 x 100 mL) and once with saturated NaCl solution (50 mL) before being dried (MgSO₄) and evaporated to dryness *in vacuo* to afford a yellow residue which was subjected to column chromatography (SiO₂, CH₂Cl₂/MeOH, 97 :3) to give 6^{A},6^{B}-dideoxy-6^{A},6^{B}-(*N*-(2-nitrophenyl)sulfonyl)aza-6^{D},6^{E}-di-*O-*(methylsulfonyl)-2^{A},2^{B},2^{C},2^{D},2^{E},2^{F},3^{A},3^{B},3^{C},3^{D},3^{E},3^{F},6^{C},6^{D},6^{E},6^{F}-tetradeca-*O*-methyl-alpha-cyclodextrin (166 mg, 77 %) as a colorless solid.
*R*_{f} (SiO₂, CH₂Cl₂/MeOH, 95:5, *v*/*v)* = 0.32; ¹H NMR (400.1 MHz, CDCl₃, 25°C): *δ* (assignment by COSY, HSQC, HMBC, ROESY): 2.91 (s, 3 H, SO₂Me), 2.96 (s, 3 H, SO₂Me), 3.08 (1H, H-6b^{B}), 3.09 (1H, H-2^{A}), 3.17 (1H, H-4^{A}), 3.37 (1H, H-4^{B}), 3.39 (s, 3 H, OMe), 3.41 (s, 3 H, OMe), 3.47 (s, 6 H, OMe), 3.48 (s, 3 H, OMe), 3.50 (7 H, H-2^{B}, H-3^{A}, H-3^{B}, H-6a^{A}, OMe), 3.51 (s, 3 H, OMe), 3.52 (s, 3 H, OMe), 3.57 (s, 3 H, OMe), 3.60 (s, 3 H, OMe), 3.61 (s, 3 H, OMe), 3.63 (s, 3 H, OMe), 3.65 (s, 6 H, OMe), 3.71 (s, 3 H, OMe), 3.87 (d, 1 H, H-6b^{A}), 4.05 (d, 1 H, ²*J*_{H6b-H6a} = 13.2 Hz, H-6a^{B}), 4.22 (t, 1 H, ²*J*_{H5-H6a} = ²*J*_{H5-H6b} = 9 Hz, H-5^{B}), 4.27 (1H, H-5^{A}), 3.04-4.48 (24 H, H-2, H-3, H-4, H-5, H-6), 5.00 (H-1^{B}) 5.00-5.03 (4 H, H-1), 5.06 (d, 1 H, ³*J*_{H1-H2} = 3.4 Hz, H-1), 7.66-7.84 (4 H, aromatic-H) ppm;
¹³C{¹H} NMR (100.6 MHz, CDCl₃, 25°C): *δ* (assignment by combined HSQC and HMBC): 36.9 (SO₂Me), 36.99 (SO₂Me), 50,12 (C-6^{B}), 54.70 (C-6^{A}), 57.78, 57.85, 58.00, 58.19, 58.34, 59.05, 59.09, 59.50, 60.87, 61.76, 61.78, 61.86, 61.92, 61.99 (OMe), 60.16 (C-5), 69.44 (C-6), 69.53 (C-5), 69.67 (C-6), 69.79 (C-5), 70.85 (C-6), 71.14 (C-6), 70.85 (C-5), 71.14 (C-5), 74.98, 79.94, 80.73, 81.20 [x2], 81.26, 81.39, 81.44, 81.53 [x2], 81.87 [x2], 82.02 [x2], 82.29, 82.35, 83.90, 86.22 (C-2, C-3, C-4), 96.65, 97.53, 99.35, 100.44, 100.63, 100.82 (C-1), 124.76, 130.61, 132.17, 132.36, 133.88, 148.14 (C arom.) ppm;
elemental analysis (%) calcd for C₅₈H₉₄N₂O₃₆S₃ (1491.55): C 46.71, H 6.35, N 1.88 found: C 46.25, H 6.36, N 1.78;
MS (ESI-TOF): *m*/*z* (%): 1513.50 (100) *[M* + Na]⁺, 1508.54 (20) [*M +* NH₄]+.

### EXAMPLE 4: Preparation of 6^{A},6^{B}-dideoxy-6^{A},6^{B}-aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-O-methyl-alpha-cyclodextrin

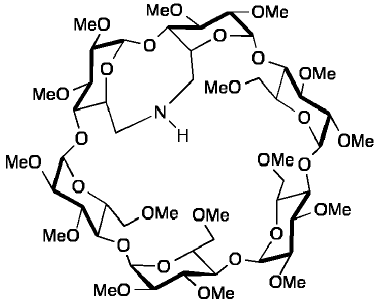

Cesium carbonate (Cs₂CO₃) (28 mg, 0.09 mmol) was added to a solution of 6^{A},6^{B}-dideoxy-*N*-((2-nitrophenyl)sulfonyl)-6^{A},6^{B}-aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (79 mg, 0.06 mmol) in 2 mL of acetonitrile (MeCN). Thiophenol (8 mg, 7.5 µL, 0.07 mmol) was added and the yellow solution stirred for 2 h at room temperature before being evaporated to dryness. The residue was dissolved in ethyl acetate (AcOEt) (50 mL) and the organic solution washed successively with 2M sodium hydroxide (NaOH) (3 × 50 mL) and saturated aqueous sodium bicarbonate (NaHCO₃) (50 mL), and dried on magnesium sulfate (MgSO₄). Finally the solvent was removed *in vacuo* and the resulting residue subjected to column chromatography (SiO₂; CH₂Cl₂/MeOH, 95:5, *v*/*v*) to afford 6^{A},6^{B}-dideoxy-6^{A},6^{B}-aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (65 mg, 96%) as a colorless solid.
*R*_{f} (SiO₂, CH₂Cl₂/MeOH, 90:10, *v*/*v*) = 0.38; ¹H NMR (500.1 MHz, CDCl₃, 25°C): *δ* (assignment by COSY) = 2.72 (dd,1H, ²*J*_{H6a-H6b} = 12.7 Hz, ³*J*_{H6a-H5} = 9.1 Hz, H-6a^{A or B}), 3.08-3.19 (6 H, H-2, H-6a^{B or A}), 3.24 (t, 1 H, ³*J*_{H4-H5} = ³*J*_{H4-H3} = 8.9 Hz, H-4^{A or B}), 3.28 (dd, 1 H, ³*J*_{H4-H5} = 10.4 Hz, ³*J*_{H4-H3} = 7 Hz, H-4^{B or A}), 3.36 (s, 6 H, OMe), 3.37 (s, 6 H, OMe), 3.45 (s, 3 H, OMe), 3.46 (s, 3 H, OMe), 3.47 (s, 3 H, OMe), 3.47 (s, 3 H, OMe), 3.48 (s, 3 H, OMe), 3.51 (s, 3 H, OMe), 3.57 (s, 3 H, OMe), 3.61 (s, 3 H, OMe), 3.62 (s, 6 H, OMe), 3.65 (s, 3 H, OMe), 3.66 (s, 3 H, OMe), 3.33-3.72 (18 H, H-2, H-3, H-4, H-5, H-6), 3.75-3.81 (3 H, H-4, H-5), 3.82 (dd, 1 H, ²*J*_{H6a-H6b} = 9.9 Hz, ³*J*_{H6a-H5} = 4.0 Hz, H-6a), 3.85-3.92 (4 H, H-4, H-5, H-6), 4.02 (t, 1 H, 3*J*_{H5-H6} =, 3*J*_{H5-H4} = 9.9 Hz, H-5), 4.94 (d, 1 H, ³*J*_{H1-H2} = 3.3 Hz, H-1), 4.96 (d, 1 H, ³*J*_{H1-H2} = 3.3 Hz, H-1), 5.00-5.03 (3 H, H-1), 5.04 (d, 1 H, ³*J*_{H1-H2} = 3.3 Hz, H-1) ppm
¹³C{¹H} NMR (125.8 MHz, CDCl₃, 25°C): *δ* (assignment by combined HSQC and HMBC) = 47.20, 54.60 (C-6^{A,B}), 57.61, 57.72 [×2], 57.77 [×2], 58.33, 58.83, 58.88 [×3], 61.35, 61.55 [×2], 61.68, 61.88, 61.98 (OMe), 70.71, 70.97, 71.08 (C-5), 71.11 (C-6), 71.16 [x2], 71.18 (C-5), 71.19, 71.27, 71.60 (C-6), 81.09, 81.14 [x2], 81.23 [x2], 81.28, 81.55, 81.59, 81.76 [×2], 81.86, 82.09, 82.15 [×2], 82.36, 83.94 [×2], 86.28 (C-2, C-3, C-4), 96.95, 99.05, 99.47, 99.57, 99.63, 99.86 (C-1) ppm;
elemental analysis (%) calcd for C₅₂H₉₁NO₂₈ (1178.28) : C 53.01, H 7.78, N 1.19 found C 53.42, H 7.81, N 1.17;
MS (ESI-TOF): *m*/*z* (%): 1178.58 (100) *[M* + H]⁺.

### EXAMPLE 5: Preparation of 6^{A},6^{B}-dideoxy-6^{A},6^{B}-aza-2^{A},2^{B},2^{C},2^{D},2^{E},2^{F},2^{G},3^{A},3^{B},3^{C},3^{D},3^{E},3^{F},3^{G},6^{C},6^{D},6^{E},6^{F},6^{G}-nonadeca-O-methyl-beta-cyclodextri n

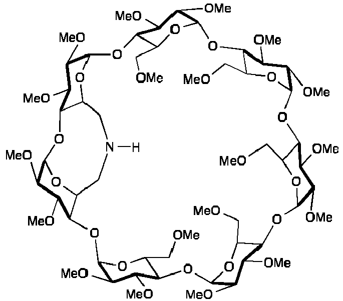

6^{A},6^{B}-dideoxy-2^{A},2^{B},2^{C},2^{D},2^{E},2^{F},2^{G},3^{A},3^{B},3^{C},3^{D},3^{E},3^{F},3^{G},6^{C}, 6^{D},6^{E},6^{F},6^{G}-nonadeca-*O*-methyl-beta-cyclodextrin (colorless solid, 90 mg, 82%) was synthesized from 6^{A},6^{B}-dideoxy-6^{A},6^{B}-N-((2-nitrophenyl)sulfonyl)-aza-2^{A},2^{B},2^{C},2^{D},2^{E},2^{F},2^{G},3^{A},3^{B},3^{C},3^{D},3^{E},3^{F},3^{G},6^{C},6^{D},6^{E},6^{F},6^{G}-hexadeca-*O-*methyl-beta-cyclodextrin (125 mg, 0.09 mmol), Cs₂CO₃ (42 mg, 0.13 mmol) and thiophenol (12 mg, 11 µL, 0.11 mmol) in acetonitrile (3mL) according to the procedure outlined in example 4.
*R*_{f} (SiO₂, CH₂Cl₂/MeOH, 90:10, *v*/*v)* = 0.36; ¹H NMR (500.1 MHz, CDCl₃, 25°C): *δ* (assignment by COSY) = 2.75 (1 H, H-6a^{A or B}), 3.12 (dd, 1 H, ³*J*_{H2-H3} = 9.8 Hz, ³*J*_{H2-H1} = 3.4 Hz, H-2), 3.15-3.21 (5 H, H-2), 3.30 (t, 1 H, ³*J*_{H4-H5} = ³*J*_{H4-H3} = 8.9 Hz, H-4^{A or B}), 3.37 (s, 9 H, OMe), 3.38 (s, 6 H, OMe), 3.47 (s, 3 H, OMe), 3.48 (s, 3 H, OMe), 3.49 (s, 3 H, OMe), 3.50 (s, 3 H, OMe), 3.51 (s, 3 H, OMe), 3.52 (s, 3 H, OMe), 3.53 (s, 3 H, OMe), 3.60 (s, 3 H, OMe), 3.62 (s, 6 H, OMe), 3.63 (s, 6 H, OMe), 3.64 (s, 3 H, OMe), 3.65 (s, 3 H, OMe), 3.33-3.68 (23 H, H-2, H-3, H-4, H-5, H-6), 3.75-3.90 (10 H, H-5, H-6), 3.99 (1 H, H-5), 4.96 (d, 1 H, ³*J*_{H1-H2} = 3.0 Hz, H-1), 5.01 (d, 1 H, ³*J*_{H1-H2} = 3.0 Hz, H-1), 5.03 (d, 1 H, ³*J*_{H1-H2} = 4.3 Hz, H-1), 5.09 (d, 1 H, ³*J*_{H1-H2} = 3.5 Hz, H-1), 5.13 (d, 1 H, ³*J*_{H1-H2} = 3.5 Hz, H-1), 5.14-5.17 (2 H, H-1) ppm;
¹³C{¹H} NMR (125.8 MHz, CDCl₃, 25°C): *δ* (assignment by combined HSQC and HMBC) = 47.48, 54.33 (C-6^{A,B}), 58.08, 58.18, 58.40 [x2], 58.43 [x2], 58.61, 58.92, 58.98 [x4], 61.16, 61.21 [x2], 61.29 [x2], 61.46, 61.55 (OMe), 70.74 [x4], 70.79 [x2], 70.99 (C-5), 71.02 [x2], 71.11, 71.22, 71.38 (C-6), 79.53, 80.05, 80.22, 80.30 [x3], 81.01, 81.40, 81.44, 81.58, 81.66, 81.77 [x3], 81.86 [x3], 82.00 [x3], 82.06 (C-2, C-3, C-4), 97.59, 98.20, 98.68, 98.87, 98.95, 99.08, 99.52 (C-1) ppm;
elemental analysis (%) calcd for C₆₁H₁₀₇NO₃₃•1.5CHCl₃ (1382.50 + 179.07) : C 48.07, H 7.00, N 0.90 found C 48.22, H 7.05, N 0.93; MS (ESI-TOF): *m*/*z* (%): 1382.67 (100) *[M* + H]⁺.

### EXAMPLE 6: Preparation of 6^{A},6^{B}-dideoxy-6^{A},6^{B}-N-(pyridin-2-ylmethyl)-aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-O-methyl-alpha-cyclodextrin

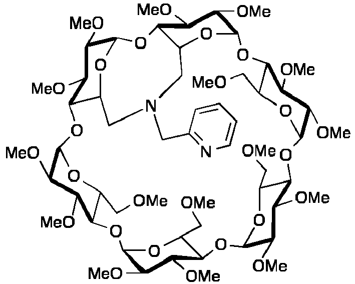

To a solution 2-pyridinecarboxaldehyde (15 mg, 13 µL, 0.14 mmol) and 6^{A},6^{B}-dideoxy-6^{A},6^{B}-aza-2^{A},2^{B},2^{C},2^{D},2^{E},2^{F},3^{A},3^{B},3^{C},3^{D},3^{E},3^{F},6^{C},6^{D}, 6^{E},6^{F}-hexadeca-*O*-methyl-alpha-cyclodextrin (150 mg, 0.13 mmol) in methanol (5 mL), were added sodium cyanoborohydride (NaBH₃CN) (10 mg, 0.16 mmol). The yellow solution was stirred for 3 h at room temperature. If necessary, extra NaBH₃CN (10 mg, 0.16 mmol) and 2-pyridinecarboxaldehyde (7 mg, 6 µL, 0.7 mmol) were added to complete the reaction. The suspension was then evaporated to dryness *in vacuo*. The residue was dissolved in dichloromethane (CH₂Cl₂) (50 mL) and the organic solution washed with aqueous 2M NaOH (3 × 50 mL) and dried (Na₂SO₄). Finally the solvent was removed *in vacuo* and the resulting residue subjected to column chromatography (SiO₂; CH₂Cl₂/MeOH, 97:3, *v*/*v)* to afford 6^{A},6^{B}-dideoxy-6^{A},6^{B}-*N*-(pyridin-2-ylmethyl)aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (137 mg, 83%) as a colorless solid.
*R*_{f} (SiO₂, CH₂Cl₂/MeOH, 90:10, *v*/*v*) = 0.56; ¹H NMR (500.13 MHz, CDCl₃, 25°C): *δ* (assignment by COSY) = 2.86 (dd, 1 H, ²*J*_{H6a-H6b} = 12.8 Hz, ³*J*_{H6-H5} = 8.8 Hz, H-6^{A or B}), 3.04 (d, 1 H, ²*J*_{H6a-H6b} = 15.7 Hz, H-6^{A or B}), 3.08 (dd, 1 H, ³*J*_{H2-H1} = 3.1 Hz, ³*J*_{H2-H3} = 10 Hz, H-2), 3.13 (s, 3 H, OMe), 3.18 (s, 3 H, OMe), 3.37 (s, 3 H, OMe), 3.40 (s, 3 H, OMe), 3.45 (s, 3 H, OMe), 3.47 (s, 6 H, OMe), 3.48 (s, 3 H, OMe), 3.50 (s, 3 H, OMe), 3.52 (s, 3 H, OMe), 3.60 (s, 3 H, OMe), 3.61 (s, 3 H, OMe), 3.62 (s, 3 H, OMe), 3.64 (s, 3 H, OMe), 3.66 (s, 3 H, OMe), 3.69 (s, 3 H, OMe), 3.10-3.88 (31 H, H-2, H-3, H-4, H-5, H-6), 3.90 (d, 1 H, ²*J* = 15.9 Hz, -C*H₂*Py), 4.01 (d, 1 H, ²*J* = 15.9 Hz, -C*H*₂Py), 4.11 (dd, 1 H, ³*J*_{H5-H4} = 9.1 Hz, ³*J*_{H5-H6} = 5.0 Hz, H-5), 4.16 (dd, 1 H, 3*J*_{H5-H4} = 10.4 Hz, ³*J*_{H5-H6} = 8.8 Hz, H-5), 4.90 (d, 1 H, ³*J*_{H1-H2} = 3.2 Hz, H-1), 4.96 (d, 1 H, ³*J*_{H1-H2} = 3.0 Hz, H-1), 5.01 (d, 1 H, ³*J*_{H1-H2} = 3.3 Hz, H-1), 5.03 (d, 1 H, ³*J*_{H1-H2} = 4.8 Hz, H-1), 5.04 (d, 1 H, ³*J*_{H1-H2} = 3.3 Hz, H-1), 5.08 (d, 1 H, ³*J*_{H1-H2} = 3.2 Hz, H-1), 7.14 (1 H, aromatic-H), 7.31 (1 H, aromatic-H), 7.60 (1 H, aromatic-H), 8.53 (1 H, aromatic-H) ppm;
¹³C{¹H} NMR (100.6 MHz, CDCl₃, 25°C): *δ* (assignment by HSQC) = 53.55 (C-6^{A or B}), 57.86, 57.90, 57.93, 58.04 [x2], 58.13, 58.85, 58.98, 59.13, 59.21 (OMe), 60.89 (-*C*H₂Py), 61.62, 61.71, 61.75, 61.89, 62.11, 62.14 (OMe), 63.87 (C-6^{A or B}), 69.80 (C-5), 71.03, 71.21 (C-6), 71.36, 71.43 (C-5), 71.45 (C-6), 71.51 (C-5), 71.61 (C-6), 71.64, 75.83 (C-5), 81.19 [x3], 81.35, 81.41, 81.50 [x2], 81.62, 82.16 [x3], 82.30, 82.45, 82.57, 82.67, 84.13, 84.22, 86.08 (C-2, C-3, C-4), 97.00, 98.74, 100.07, 100.21, 100.26, 100.44 (C-1), 121.78, 121.91, 136.20, 149.35, 160.52 (C aromatic) ppm; elemental analysis (%) calcd for C₅₈H₉₆N₂O₂₈•0.66 CH₂Cl₂ (1269.39 + 56.05): C 53.14, H 7.40, N 2.11 found: C 53.12, H 7.31, N 2.07;
MS (ESI-TOF): *m*/*z* (%): 1269.63 (100) *[M* + H]⁺.

### EXAMPLE 7: Preparation of 6^{A},6^{B}-dideoxy-6^{A},6^{B}-N,N-dimethylammonio-2^{A},2^{B},2^{C},2^{D},2^{E},2^{F},3^{A},3^{B},3^{C},3^{D},3^{E},3^{F},6^{C},6^{D},6^{E},6^{F}-hexadeca-O-methyl-alpha-cyclodextrin chloride

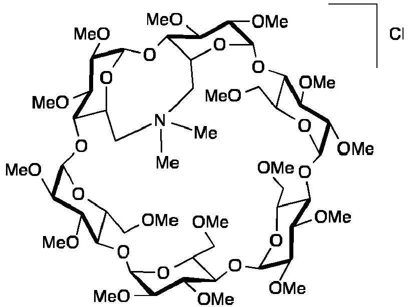

Silver(I) oxide (Ag₂O) (172 mg, 0.74 mmol) was added to a solution of 6^{A},6^{B}-dideoxy-6^{A},6^{B}-aza-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin (132 mg, 0.094 mmol) in dimethylformamide (DMF) (3.5 mL). Methyl iodide (132 mg, 58 µL, 0.94 mmol) was then added dropwise and the suspension was stirred for 24 h at room temperature before being evaporated to dryness. The reaction mixture was retaken in chloroform (CHCl₃)(50 mL) and filtered over Celite. The filtrate was washed successively with saturated aqueous NaCl (3 x 50 mL) before being dried (NaCl) and evaporated to dryness *in vacuo*. The residue was subjected to column chromatography (SiO₂, CH₂Cl₂/MeOH, 90/10) to afford a yellow residue, which was dissolved in CHCl₃ before being washed with saturated aqueous NaCl (3 x 75 mL). The organic extract was then dried (NaCl) before being evaporated to dryness to afford 6^{A},6^{B}-dideoxy-6^{A},6^{B}-*N,N*-dimethylammonio-2^{A},2^{B},2^{C},2^{D},2^{E},2^{F},3^{A},3^{B},3^{C},3^{D},3^{E},3^{F},6^{C},6^{D},6^{E},6^{F}-hexadeca-*O*-methyl-alpha-cyclodextrin chloride (110 mg, 95%) as a colorless solid.
¹H NMR (300.1 MHz, CDCl₃, 25°C): *δ* (assignment by COSY, HMBC, HSQC, ROESY) = 3.22 (s, 3 H, NMe), 3.36 (s, 3 H, OMe), 3.37 (s, 3 H, OMe), 3.39 (s, 3 H, OMe), 3.45 (s, 3 H, OMe), 3.46 (s, 6 H, OMe), 3.47 (s, 3 H, OMe), 3.48 (s, 9 H, OMe), 3.56 (s, 3 H, OMe), 3.59 (s, 3 H, OMe), 3.61 (s, 6 H, OMe), 3.62 (s, 3 H, OMe), 3.63 (s, 3 H, OMe), 3.66 (s, 3 H, NMe), 3.04-3.87 (31 H, H-2, H-3, H-4, H-5, H-6), 3.98 (t, 1 H, 3*J*_{H5-H6} = 3*J*_{H5-H4} = 9.9 Hz, H-5^{A or B}), 4.00 (1H, H-6^{A or B}), 4.18 (d, 1 H, ²*J*_{H6a-H6b} = 14 Hz, H-6), 4.32 (dd, 1 H, ³*J*_{H5-H4} = 8.6 Hz, ³*J*_{H5-H6} = 3.6 Hz, H-5^{A or B}), 4.94 (1 H, H-6^{A or B}), 4.89-5.03 (5 H, H-1), 5.10 (d, 1 H, ³*J*_{H1-H2} = 4.2 Hz, H-1) ppm;
¹³C{¹H} NMR (125.8 MHz, CDCl₃, 25°C): *δ* (assignment by combined HSQC and HMBC) = 48.49 (NMe), 58.05 [x2], 58.17, 58.26, 58.30, 58.39, 59.15 [×3], 59.63 (OMe), 60.62 (NMe), 61.41, 61.57, 61.65, 61.78 [x2], 62.28, 63.30 (OMe), 63.30 (C-5), 66.69 (C-6), 68.93 (C-5), 71.55 (C-6), 71.71 [x2] (C-5), 71.74 71.83 [×2] (C-6), 71.99, 72.09 (C-5), 73.82 (C-6) 80.10, 80.57, 80.68, 80.92, 81.36 [x3], 81.41, 81.77, 82.14, 82.19, 82.23, 82.40, 82.61, 83.58, 83.62, 83.91[x2] (C-2, C-3, C-4), 96.87, 98.64, 99.67, 100.01, 100.30, 101.00 (C-1) ppm;
elemental analysis (%) calcd for C₅₄H₉₆ClNO₂₈ (1241.58): C 52.19, H 7.79, N 1.13 found: C 51.88, H 8.14, N 0.79; MS (ESI-TOF): *m*/*z* (%): 1206.61 (100) [M - Cl]⁺.

### EXAMPLE 8: Preparation of 6^{A},6^{B}-dideoxy-6^{A},6^{B}-N,N-dimethylammonio 2^{A},2^{B},2^{C},2^{D},2^{E},2^{F},2^{G},3^{A},3^{B},3^{C},3^{D},3^{E},3^{F},3^{G},6^{C},6^{D},6^{E}, 6^{F},6^{G}-nonadeca-O-methyl-beta-cyclodextrin chloride

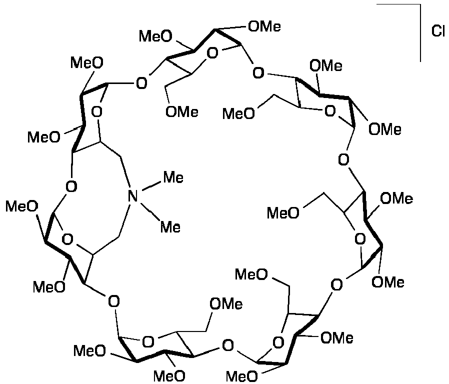

6^{A},6^{B}-dideoxy-6^{A},6^{B}-*N,N*-dimethylammonio-2^{A},2^{B},2^{C},2^{D},2^{E},2^{F},2^{G},3^{A},3^{B}, 3^{C},3^{D},3^{E},3^{F},3^{G},6^{C},6^{D},6^{E},6^{F},6^{G}-*O*-methyl-beta-cyclodextrin chloride (colorless solid, 564 mg, 90 %) was synthesized from 6^{A},6^{B}-dideoxy-6^{A},6^{B}-aza-2^{A},2^{B},2^{C},2^{D},2^{E},2^{F},2^{G},3^{A},3^{B},3^{C},3^{D},3^{E},3^{F},3^{G},6^{C},6^{D},6^{E},6^{F},6^{G}-nonadeca-*O*-methyl-beta-cyclodextrin (0.6 g, 0.43 mmol), iodomethane (0.61 g, 266 µL, 4.34 mmol) and silver oxide (804 mg, 3.47 mmol) in N,N-dimethylformamide (16.5 mL) according to the procedure outlined in example 7.
¹H NMR (500.1 MHz, CDCl₃, 25°C): *δ* ¹H NMR (300.1 MHz, CDCl₃, 25°C): *δ* (assignment by COSY, HMBC, HSQC and ROESY) = 3.36 (s, 3 H, NMe), 3.36 (s, 3 H, OMe), 3.37 (s, 3 H, OMe), 3.39 (s, 3 H, OMe), 3.46 (s, 12 H, OMe), 3.48 (s, 9 H, OMe), 3.53 (s, 3 H, OMe), 3.57 (s, 3 H, OMe), 3.58 (s, 3 H, OMe), 3.59 (s, 9 H, OMe), 3.61 (s, 3 H, OMe), 3.65 (s, 3 H, NMe), 3.12-3.93 (39 H, H-2, H-3, H-4, H-5, H-6), 4.10 (d, 1H, ²*J*_{H6a-H6b} = 14 Hz, H-6^{A or B}), 4.35 (dd, 1 H, ³*J*_{H5-H4} = 7.3 Hz, ³*J*_{H5-H6} = 3.0 Hz, H-5^{A or B}), 4.69 (dd, 1 H, ²*J*_{H6a-H6b} = 15.4 Hz, 3*J*_{H5-H6} = 3.5 Hz, H-6^{A or B}), 4.88 (d, 1 H, ³*J*_{H1-H2} = 3.3 Hz, H-1), 5.00 (d, 1 H, ³*J*_{H1-H2} = 3.3 Hz, H-1), 5.06 (3 H, H-1), 5.10 (d, 1H, ³*J*_{H1-H2} = 3.4 Hz, H-1) ppm;
¹³C{¹H} NMR (125.8 MHz, CDCl₃, 25°C): *δ* (assignment by HSQC and HMBC) = 48.18 (NMe), 57.17, 57.26, 57.32, 57.37, 57.55, 57.63, 58.0, 58.1 [x3], 58.5 (OMe), 59.41 (NMe), 59.64, 59.84, 60.01, 60.14, 60.31, 60.36, 61.15, 62.35 (OMe), 65.78 (C-6), 68.63, 69.66 (C-5), 69.92 (C-6), 70.19 (C-5), 70.37 (C-6), 70.50, 70.97 (C-5), 71.05, 72.86 (C-6), 75.82, 79.36 (C-5) 78.92, 79.48, 79.52, 79.63, 79.90, 80.02, 80.28, 80.46, 80.55 [x3], 80.68, 80.74, 81.13, 81.19, 82.16, 82.91, 83.32 (C-2, C-3, C-4), 95.19, 96.04, 98.27, 98.57, 98.60, 99.25, 99.85 (C-1) ppm;
elemental analysis (%) calcd for C₆₃H₁₁₂ClNO₃₃ + 2 H₂O (1447.01 + 36.03): C 51.02, H 7.88, N 0.94 found: C 50.70, H 7.51, N 0.92;
MS (ESI-TOF): *m*/*z* (%): 1410.72 (100) [*M -* Cl]⁺.

## Claims

1. Rigidified cyclodextrin derivatives comprising a core formed of a cyclodextrin unit wherein
a. two glucose units, whether adjacent or not, are linked through a -N(R¹R²)- bridge attached to the C-6 carbon atoms of the bridged glucose units,
b. the C-6 carbon atom of glucose units which are not bound to the said bridge are substituted by R³ residues, and
c. the C-2 and C-3 carbon atoms are substituted by OR⁴ residues,
the said derivative being of formula (I) 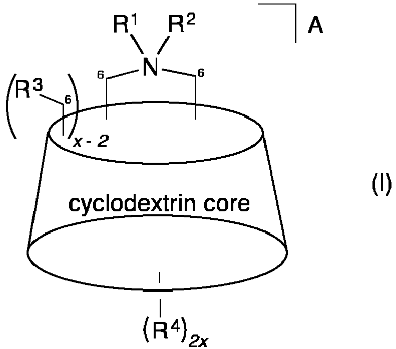 wherein,
"cyclodextrin core" represents the moiety obtained by removal of all -CH₂OH groups and all hydrogen atoms of the hydroxyl groups attached to the C-2 and C-3 carbon atoms of the glucose units of alpha-cyclodextrin, beta-cyclodextrin or gamma-cyclodextrin responding to the following formula, 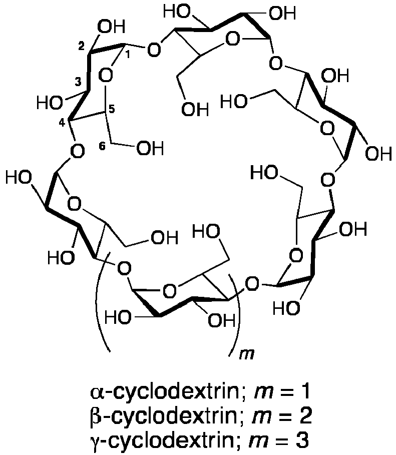
x is an integer comprised between 6 and 8,
R¹, optionally linked to R², is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
R², optionally absent, is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
the R³ residues, identical or different, optionally linked together or linked to the R¹ or R² residues, are chosen from hydrogen, the hydroxyl radical and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
R⁴, identical or different, optionally linked together, are chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
A, is an anion, preferentially selected from Cl⁻, Br⁻, I⁻, BF₄⁻, PF₆⁻, B(aryl)₄⁻ CF₃SO₃⁻, AcO⁻, CF₃CO₂⁻, PO₄⁻, SO₄⁻, picrate, phosphonate, or a mixture thereof, the said anion or mixture of anions having a total charge ranging from 0 to 20, preferentially from 0 to 4,
with the proviso that the compounds of formula (a) are excluded

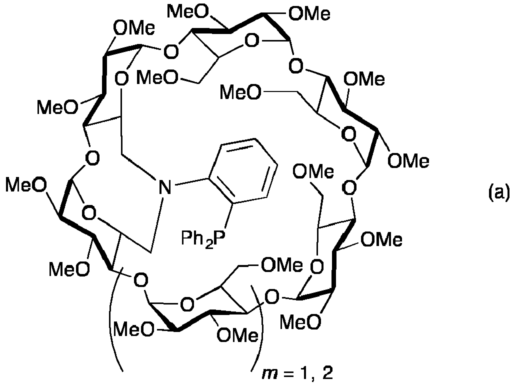

2. Process for the preparation of derivatives of formula (I) according to claim 1 comprising the following steps:
a) reacting a cyclodextrin derivative of formula (II) 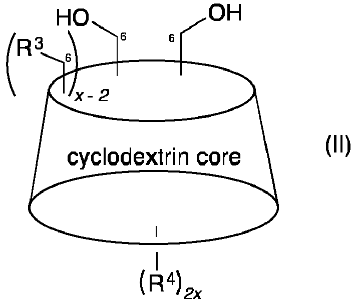 wherein R³, R⁴ and x are as defined in claim 1,
with 2-nitrobenzenesulfonamide under Mitsonobu conditions, so as to obtain the cyclodextrin derivative of formula I in which R¹ is a 2-nitrobenzenesulfonyl group and wherein R² is absent,
b) hydrolyzing the sulfonyl derivative obtained in step a) to yield a derivative of formula (I) wherein R¹ is H and where R² is absent,
c) reacting the derivative obtained in step b), in the presence of a reducing agent, preferably sodium cyanoborohydride, with an aldehyde of formula (III)
R⁵-CHO (III)
in which R⁵ is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
to afford a tertiary amine of formula (I) wherein R¹ is defined as in claim 1 and where R² is absent.

3. Process for the preparation of derivatives of formula (I) comprising the step of reacting a cyclodextrin derivative of formula IV : 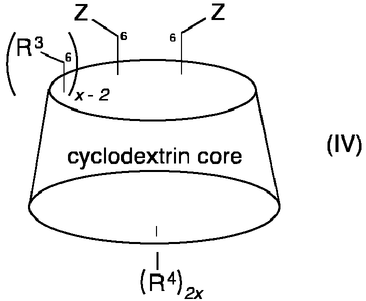 wherein R³, R⁴ and x are as defined in claim 1, and wherein the Z residues, identical or different and attached to C-6 carbon atoms of either adjacent or non adjacent glucose units, are leaving groups, preferentially p-toluene sulfonyloxy, methylsulfonyloxy, trifluoromethylsulfonyloxy, chloro, bromo or iodo,
with 2-nitrobenzenesulfonamide in the presence of a carbonate, preferentially selected from the group comprising Li₂CO₃, Na₂CO₃, K₂CO₃, and Cs₂CO₃, so as to obtain the cyclodextrin derivative of formula (I) wherein R¹ is the 2-nitrobenzenesulfonyl group, and R² is absent.

4. Process comprising the step of reacting a derivative of formula (I) wherein R¹, R³, R⁴, A, x and y are as defined in claim 1, and wherein R² is absent,
with a R²X reagent, wherein R² is chosen from hydrogen and saturated or unsaturated, optionally aromatic, linear, branched or cyclic C₁-C₁₀₀ carbon-based radicals optionally comprising 1 to 50 heteroatoms chosen from O, N, S, F, Cl, Br, I, Si, and P,
and wherein X is a leaving group, preferentially *p*-toluene sulfonyloxy, methylsulfonyloxy, trifluoromethylsulfonyloxy, chloro, bromo or iodo,
for the preparation of a derivative of formula (V) 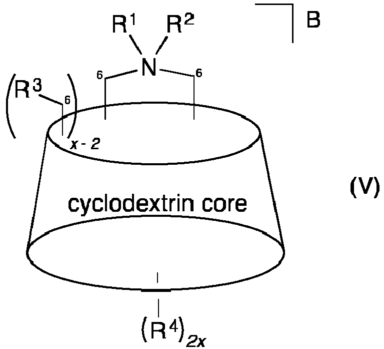 wherein R¹, R², R³, R⁴, x and y are as defined in claim 1 and B is an anion or a mixture of anions of a total charge superior to that of A.

5. Separation material for applications in chromatography comprising a rigidified cyclodextrin derivative according to claim 1 grafted on a support.

6. Method for protecting colored cellulosic fibers involving grafting of derivatives according to the invention on the said fibers.

7. A complex for targeted drug delivery comprising a rigidified cyclodextrin derivative according to the invention and a therapeutic agent.
